# EUROPÄISCHE PATENTSCHRIFT

(11) **EP 4 057 919 B1**
(45) Veröffentlichungstag und Bekanntmachung des Hinweises auf die Patenterteilung: **01.04.2026**
(21) Anmeldenummer: 20807313.0
(22) Anmeldetag: 12.11.2020
(51) Int. Cl.: A61B 17/16

(54) **CHIRURGISCHER FRÄSER MIT VERBESSERTER SPANABFUHR**
SURGICAL MILLING CUTTER WITH IMPROVED CHIP REMOVAL
FRAISE CHIRURGICALE À ÉLIMINATION AMÉLIORÉE DES COPEAUX

(30) Priorität: 13.11.2019 DE 102019130568
(43) Veröffentlichungstag der Anmeldung: 21.09.2022
(73) Patentinhaber: Aesculap AG, 78532 Tuttlingen (DE)
(72) Erfinder: BLUST, Edgar, 78126 Königsfeld (DE); MACHILL, Martin, 78604 Rietheim-Weilheim (DE)
(74) Vertreter: Winter, Brandl - Partnerschaft mbB
(86) Internationale Anmeldenummer: PCT/EP2020/081822
(87) Internationale Veröffentlichungsnummer: WO 2021/094411

(56) Entgegenhaltungen:
- EP-A2- 0 925 760
- WO-A1-2011/023381
- KR-B1- 101 200 448
- US-A- 5 788 699
- US-A1- 2013 261 628

## Beschreibung

Die vorliegende Erfindung betrifft einen chirurgischen Fräser mit einem Schaft zum drehangetriebenen Koppeln mit einer Antriebseinheit um eine sich in longitudinaler Richtung des Schafts erstreckende Rotationsachse und einem distal an dem Schaft angeordneten Fräserkopf, der zumindest zwei Zähne mit Schneiden zur rotatorisch abtragenden Bearbeitung von Gewebe aufweist, die jeweils zur abtragenden Bearbeitung von Gewebe sowohl in distaler Richtung als auch in lateraler Richtung ausgebildet sind und sich von distal an der Rotationsachse des Fräsers beginnend in Richtung proximal nach radial außen erstrecken, wobei jeweils zwischen in umfänglicher Richtung benachbarten Zähnen ein Spanraum als Freiraum ausgebildet ist.

Chirurgische Fräser sind aus dem Stand der Technik allgemein bekannt und dienen einer abtragenden Bearbeitung von hartem Gewebe wie zum Beispiel Knochen oder Knorpel, indem sie rotatorisch um eine in ihrer Längsrichtung verlaufenden Rotationsachse angetrieben werden. Sogenannte Rosenbohrer oder Rosenfräser sind Fräswerkzeuge mit einem annähernd kugelförmigen Fräserkopf, der mit vielen, Schneiden tragenden Zähnen versehen ist, je nach Durchmesser sechs bis 14 Zähne. Die Zähne und deren Schneiden laufen am distalen Endbereich des Fräserkopfs an der Rotationsachse des Fräsers zusammen. Infolge der hohen Anzahl an Zähnen nimmt deren Höhe in Richtung des distalen Endbereichs ab und ist unmittelbar an der Rotationsachse Null. Damit existiert unmittelbar an und in der Nähe des distalen Endes des Fräsers kein Zahnzwischenraum zwischen den einzelnen Zähnen. Durch eine entsprechende geometrische Optimierung kann zwar ggf. im Bereich des distalen Endes des Fräserkopfs eine etwas größere Zahnhöhe und damit etwas größere Spanräume geschaffen werden. Da sich die Schneiden allerdings im Zentrum, d.h. an der Drehachse des Fräskopfs treffen, bestehen auch im Falle einer solchen Optimierung die Nachteile einer schwierigen Spanabfuhr, einer dadurch bedingten relativ hohen Erwärmung sowie einer stark eingeschränkten oder nicht vorliegenden Sicht auf den Situs.

Die Zähne / Schneiden derartiger Rosenfräser werden üblicherweise durch Schleifen hergestellt. Dabei werden sogenannte Spitzscheiben verwendet. Diese weisen üblicherweise einen Schleifwinkel von 60°bis 90° auf und bilden durch ihren Materialabtrag im Fräserkopf den Zahnzwischenraum aus. Die Eindringtiefe der Spitzscheibe in den Fräserkopf wird derart eingestellt, dass die Zahnbrust eines vorherigen Zahnzwischenraumes mit dem Zahnrücken eines folgenden Zahnzwischenraums einen Zahn mit einer entsprechenden Schneide bildet. Üblicherweise werden die Zähne mit einem Drall, also wendelförmig, in den Fräserkopf eingebracht, damit die Spitzscheibe beim Schleifen des proximalen Bereichs des Fräserkopfs nicht in den Fräserschaft einläuft. So können die Zähne bzw. die Schneiden im proximalen Bereich des Fräserkopfs etwas näher zur Rotationsachse hin ausgebildet werden. Die Spitzscheiben sind so eingestellt, dass der Spanwinkel der Schneiden negativ ausgebildet wird und meist zwischen -10° bis -40° beträgt. Der Freiwinkel an den Schneiden ist entsprechend sehr groß und beträgt zwischen 40° und 70°.

Ein in Bezug auf die Herstellung vorliegender Nachteil von solchen Rosenfräsern ist die durch deren hohe Anzahl an Zähnen / Schneiden bedingte lange Laufzeit der Schleifmaschine bei der Herstellung, was zu hohen Herstellkosten führt.

Ein in Bezug auf die praktische Verwendung vorliegender Nachteil dieser Art von Fräswerkzeugen ist, dass durch die herstellungsbedingt relativ spitzen Zähne und deren große Anzahl bei einer Bearbeitung von Knochen oft ein sogenanntes "Rattern" auftreten kann, was sich durch unkontrolliertes Wegspringen von der zu bearbeitenden Stelle zeigt, so dass präzises Arbeiten nicht oder nur ausgesprochen schwierig möglich ist.

Ein weiterer Nachteil ist durch den relativ kleinen als Spanraum wirkenden Zahnzwischenraum zwischen den Zähnen im distalen Endbereich bedingt. Bei einer Bearbeitung abgetragenes Knochenmaterial sammelt sich im Spanraum und muss von dort abtransportiert werden, um für neu abgetragenes Material Raum zu schaffen. Ist der Materialtransport durch die Zahnzwischenräume unzureichend, kommt es zu Verstopfungen des Spanraums und zu einer Abschirmung der Schneiden durch im Spanraum angesammeltes Material. Im Ergebnis ist weiterer Materialabtrag nicht mehr möglich. Dies ist einer der Gründe, warum es bei derartigen Fräserköpfen problematisch ist, eine zufriedenstellende Schneidperformance und Schneidleistung in axialer Richtung zu bewirken. Dieser Nachteil ist besonders schwerwiegend, da bei chirurgischen Eingriffen sehr häufig in der Tiefe (also axial) gearbeitet werden muss, um in axialer Richtung Knochen abzutragen. Eine im Zentrum des Fräserkopfs verstopfte Schneide bzw. Zahnzwischenraum ist vom Operateur nur schlecht oder nicht zu erkennen, da ihm die Sicht durch den Fräserkopf selbst versperrt ist. Eine Reinigung des verstopften Zahnzwischenraumes ist während einer OP sehr schwer zu bewerkstelligen. Ist erst der Zahnzwischenraum verstopft und bearbeitet der Operateur dann auch noch weiter Knochen, kann es zu erhöhten Andrückkräften und erhöhter Reibung kommen, was eine Temperaturerhöhung mit sich zieht. Dies ist besonders kritisch, da es bereits bei Temperaturen oberhalb von nur 45°C zu einer Koagulation von Eiweiß des Patienten mit bekannten nachteiligen Folgen kommen kann.

Um die letztgenannte Problematik zu mindern, erfolgt bei der Anwendung derartiger Fräser ein Spülen bzw. Kühlen mit Flüssigkeit. Die Flüssigkeit unterstützt die Spanabfuhr und reduziert die Erwärmung an der zu bearbeitenden Stelle. Allerdings kann es bei nicht ausreichender Schneidperformance und Zuführung von Flüssigkeit punktuell zu erhöhter Erwärmung kommen, was zur Koagulation des bearbeiteten Knochens führen kann. Außerdem kann es zu übermäßiger Erwärmung des im Zahnzwischenraum befindlichen Materials kommen, das dort infolge der Erwärmung koagulieren kann und sich nur sehr schwer wieder entfernen lässt.

Ein weiterer Nachteil kann darin bestehen, dass infolge der Geometrie des Fräsers für einen Chirurgen keine Sichtmöglichkeit auf den Situs, d.h. das Operationsgebiet im Bereich der Werkzeugspitze, besteht. Dies trifft sowohl bei im Betrieb als auch bei im Stillstand befindlichen Fräser zu. Zur Begutachtung des Situs ist daher zumindest ein teilweises, wenn nicht sogar vollständiges Entfernen des Fräsers aus dem Operationsbereich erforderlich.

Schließlich sind die vielen tiefen Zahnzwischenräume derartiger Rosenfräser oder Rosenbohrer im klinischen Alltag in Rahmen ihrer Aufbereitung nur schwer zu reinigen.

Neben den vorstehend beschriebenen Fräsern sind in der Medizintechnik Fräswerkzeuge mit zwei Schneiden, zum Beispiel sogenannte Olivenfräser oder Neuro-Cutter, sowie sogenannte Twin-Cutter-Fräswerkzeuge bekannt. Diese weisen jedoch einen deutlich geringeren Spanraum auf.

Einen relativ großen Spanraum weist ein mit lediglich einer Schneide versehener chirurgischer Fräser auf, ein sogenannter Einschneider. Ein Beispiel für einen solches Instrument offenbart die US 2017 015 0974 A1. Durch das Vorsehen lediglich einer Schneide und eine zur Rotationsachse unsymmetrische /exzentrische Gestaltung des Fräserkopfs ist dessen Masse allerdings exzentrisch verteilt, so dass im Betrieb eine Unwucht entsteht. Dies führt zu einem rauen, unruhigen Lauf des Fräswerkzeugs, wodurch das Handstück und besonders dessen Lagerungen stark belastet werden und es zu erhöhten Laufgeräuschen kommt.

Aus der WO 2011 / 023 381 A1 und der KR 101 200 448 B1 sind jeweils medizinische Fräser mit gewendelten Zähnen bekannt. Die US 2013 / 261 628 A1 offenbart einen Bohrer für eine Bohrmaschine. Auch bei dem Bohrer sind die Schneideflächen gewendelt.

Die EP 0 925 760 A2 und die US 5 788 699 A zeigen jeweils medizinische Bohrer. Der Bohrer der EP 0 925 760 A2 eignet sich aber nicht zum axialen Abtragen von Gewebe. Der Bohrer, der in der US 5 788 699 A offenbart ist, weist keine Punktsymmetrie bezüglich einer Rotationsachse auf. Die Schneiden des Bohrers sind vielmehr auf derselben Seite der Rotationsachse angeordnet. Dadurch kann beim Bohren oder bei Rotation um die Rotationsachse eine Unwucht auftreten.

Vor diesem Hintergrund liegt der vorliegenden Erfindung die Aufgabe zugrunde, die genannten Nachteile des Stands der Technik zu verringern, insbesondere einen Fräser mit einer in axialer Richtung verbesserten Schneidleistung, geringer Wärmeproduktion sowie verbesserter Spanabfuhr aus dem distalen Endbereich bereit zu stellen, insbesondere für Highspeed-Anwendungen in der Medizin zum Bearbeiten von Knochen. Eine weitere Aufgabe besteht darin, einen besonders gut zu reinigenden Fräser bereitzustellen, der insbesondere für eine Gewinnung von Gewebematerial wie Knochen oder Knorpel zur weiteren künstlichen Materialgewinnung geeignet ist. Schließlich soll die Erfindung ein verbessertes Verfahren zur Gewinnung von Knochenmaterial und/oder Knorpelmaterial zur Verfügung stellen.

Diese Aufgabe wird nach der vorliegenden Erfindung gelöst durch einen chirurgischen Fräser nach Anspruch 1, also einen chirurgischen Fräser mit einem Schaft zum drehangetriebenen Koppeln mit einer Antriebseinheit um eine sich in longitudinaler Richtung des Schafts erstreckende Rotationsachse, insbesondere mit einem chirurgischen Handstück, und einem distal an dem Schaft angeordneten Fräserkopf, wobei der Fräserkopf zumindest zwei Zähne jeweils mit Schneiden zur rotatorisch abtragenden Bearbeitung von Gewebe aufweist, wobei die Schneiden jeweils zur abtragenden Bearbeitung von Gewebe sowohl in axialer und/oder distaler Richtung als auch in radialer und/oder lateraler Richtung ausgebildet sind, wobei jeweils zwischen in umfänglicher Richtung benachbarten Zähnen ein Spanraum als Freiraum, auch als Zahnzwischenraum bezeichnet, ausgebildet ist. Jeder Spanraum erstreckt sich auf der der jeweiligen Schneide zugewandten Seite der Rotationsachse von der Schneide bis in einen Bereich auf der der entsprechenden Schneide abgewandten Seite der Rotationsachse, zumindest in einem distalen Abschnitt des Fräsers. Die Zähne sind quer zur Rotationsachse unversetzt einander diametral gegenüberliegend in einer Ebene angeordnet und ausgebildet. Die Zähne können insbesondere derart ausgebildet sein, dass eine Brustfläche des einen Zahns und eine Rückenfläche des anderen Zahns gemeinsam eine ebene Fläche ausbilden, in der die Rotationsachse liegt.

Man kann auch sagen, dass nach der Erfindung jeder Spanraum schneidenseitig der Rotationsachse des Fräsers ausgebildet ist und sich zumindest in einem distalen Bereich auf die von der jeweiligen Schneide abgewandte Seite der Rotationsachse erstreckt. Der erfindungsgemäße chirurgische Fräser ist vorzugweise rotationssymmetrisch zu seiner Rotationachse und/oder mit zur Rotationsachse symmetrischer Massenverteilung ausgebildet, so dass er unwuchtfrei betrieben werden kann.

Die Rückenfläche des einen Zahns und die Brustfläche des anderen Zahns können gemeinsam eine weitere ebene Fläche ausbilden, die der vorgenannten Fläche gegenüberliegt. Die beiden Flächen können zueinander parallel sein. Vorzugsweise sind sie in einer Richtung quer zur Rotationsachse zueinander parallel und in Richtung der Rotationsachse jedoch zueinander um einen Zahndickenwinkel geneigt.

Es ist ein besonderer Vorteil der Erfindung, dass die Gestaltung der Zähne bzw. der Schneiden derart ist, dass der Zahnzwischenraum / Spanraum im distalen Endbereich des Fräserkopfs nahe der oder an der Rotationsachse (an der Spitze) möglichst groß ist. Dadurch wird eine gute Abfuhr des mittels des Fräsers abgetragenen Materials (insbesondere Knochenmehl oder -späne) erreicht. Eine flüssigkeitsbasierte Unterstützung zur Materialabfuhr kann grundsätzlich in den meisten Fällen in vorteilhafter Weise reduziert werden. Eine Koagulation des bearbeiteten Knochens sowie außerdem des im Zahnzwischenraum befindlichen abgetragenen Materials infolge übermäßiger Erwärmung kann sicher vermieden werden. Daher und infolge der guten Zugänglichkeit der Zähne, Zahnzwischenräume und Schneiden ist der Fräser besonders einfach und gut zu reinigen. Ein weiterer besonderer Vorteil der Erfindung liegt darin, dass dem Chirurgen infolge der erfindungsgemäßen Gestalt des Fräsers und dessen Spanräume eine besonders gute Sicht auf den Situs ermöglicht wird, ohne dass dazu ein vollständiges oder teilweises Entfernen des Fräsers aus dem Operationsbereich erforderlich ist. Schließlich ist die Wärmeerzeugung ausgesprochen gering, so dass eine unverhältnismäßige Erwärmung, insbesondere auf Temperaturen von 45°C und mehr, von Knochenmaterial bei der Bearbeitung mit dem erfindungsgemäßen Fräser sicher vermieden werden kann. Vorteilhafte Folge davon kann sein, dass abgetragenes Knochenmaterial nicht geschädigt wird / ist und daher für eine weitere Verwendung, zum Beispiel eine Zucht von Knochenmaterial, genutzt werden kann.

Vorteilhafte Ausführungsformen der Erfindung sind in den Unteransprüchen beansprucht und werden nachfolgend näher erläutert.

Eine Ausführungsform ist dadurch gekennzeichnet, dass die Schneiden jeweils bogenförmig von distal-radial-innen nach proximal-radial-außen ausgebildet sind. Sie können außerdem nahezu halbkreisbogenförmig ausgebildet sein und/oder von der in radialer Richtung breitesten Stelle des Fräserkopfs bogenförmig zurück nach radial innen zum Fräserschaft verlaufen. Vorzugsweise erstrecken sich die Schneiden des Fräserkopf von distal an der Rotationsachse des Fräsers beginnend in Richtung proximal nach radial außen. Die Schneiden jedes Fräserkopfs können insbesondere randseitig ausgebildet und derart geformt sein, dass sie bei einer Rotation des Fräsers um seine Rotationsachse (Längsachse) eine Hüllbahn von vorzugsweise im Wesentlichen kugelförmiger Form ablaufen. Ein solcher Fräser kann auch als Kugelfräser bezeichnet werden und eignet sich besonders gut für Verwendungen, bei denen in lateraler / radialer und/oder axialer / distaler Richtung Material abzutragen ist. Im Rahmen der Erfindung kann der Fräserkopf außerdem derart ausgebildet sein, dass die durch die Schneiden bei einer Rotation des Fräsers beschriebene Hüllgeometrie andere Formen besitzt, zum Beispiel die eines Flammenfräsers, Kegelfräsers, Walzenfräsers oder Ovalfräsers.

Nach einer weiteren Ausführungsform können die Zähne bzw. die Schneiden jeweils ungewendelt und/oder drallfrei ausgebildet sein. Dies verbessert die Zugänglichkeit der Zahnzwischenräume und damit die Reinigungseignung. Außerdem sind derartige Zahn-/Schneidenformen relativ einfach, schnell und kostengünstig herstellbar.

Nach einer weiteren Ausführungsform kann der Fräser genau zwei Zähne bzw. Schneiden aufweisen, die auf einander diametral gegenüberliegenden Seiten der Rotationsachse des Fräsers angeordnet sind. Die Schneiden sind auf den voneinander abgewandten Seiten der beiden Zähne ausgebildet, so dass beide Schneiden bei einer Rotation des Fräsers um seine Rotationsachse einen Materialabtrag bewirken. Die schneidenseitige Fläche des Zahns wird auch als Zahnbrust bezeichnet, die von der Schneide abgewandte Seite des Zahns als Zahnrücken. Der jeweilige schneidenseitige Freiraum (also der auf der Seite der Zahnbrust) stellt den Spanraum in Sinne der Erfindung dar für mit der entsprechenden Schneide abgetragenes Material. Durch die beschriebene Anordnung und Ausbildung der Schneiden / Zähne sind die beiden Zahnzwischenräume besonders groß, so dass ein besonders guter Abtransport von abgetragenem Material sichergestellt ist und eine schnelle Heilung bei nicht koagulierten Knochen erzielt werden kann.

Eine solche zweizahnige oder zweischneidige Ausbildung kann besonders effektiv und einfach bewirkt werden, indem der Fräserkopf jeweils auf beiden Seiten der Rotationsachse (lateral) zumindest am distalen Ende des Fräsers bis auf die Rotationsachse abgetragen ist. Diese Abtragung bildet dann eine in weiten Teilen vorzugsweise ebene Fläche, die die Zahnbrust des jeweiligen Zahns ausbildet.

Alternativ oder zusätzlich kann sich eine Stufe oder Schulter beginnend distal an der Rotationsachse in Richtung proximal erstrecken, so dass die die Zahnbrust ausbildende Fläche mit einer Stufe oder Schulter in den Zahnrücken des gegenüberliegenden Zahns übergeht. Diese Stufe oder Schulter bildet eine Begrenzung zwischen der Zahnbrust eines Zahns und der in Schnittrichtung hinteren Fläche des benachbarten Zahns, also dem Zahnrücken des anderen Zahns, aus. Bei einer im Hinblick auf die Herstellung besonders vorteilhaften Ausführungsform verläuft die Stufe oder Schulter von der Spitze des Fräserkopfs, als von dem Schnittpunkt des distalen Endes des Fräserkopf mit der Rotationsachse, in einem gegenüber der Rotationsachse schrägem Winkel, so dass im hinteren (proximalen) Bereich des Fräserkopfs der Schaft des Fräsers gerade eben nicht tangiert wird. Dieser Winkel liegt vorzugsweise in der Ebene der jeweiligen Brustfläche, wird auch als Einstellwinkel bezeichnet und liegt vorzugsweise in einem Bereich von 2° bis 10°. Anders ausgedrückt bildet die Zahnbrust zusammen mit dem umlaufenden Freiwinkel die Schneide oder Schneidkante. Alternativ oder zusätzlich kann der Zahnrücken mit einer konstanten Breite ausgebildet sein.

Die Zahnbrust ist derart ausgebildet und/oder angeordnet, dass der Spanwinkel an der Schneide 0° beträgt.

Eine im Hinblick auf die Materialabfuhr besonders vorteilhafte Ausführungsform der Erfindung ist dadurch gekennzeichnet, dass zumindest eine der Schneiden wenigstens eine Unterbrechung oder Nut, insbesondere als Spanbrechernut, aufweist. Diese kann insbesondere in zentripetaler Richtung in den Zahn eingebracht sein. Durch die Unterbrechung der Scheiden wird deren Breite reduziert, so dass ein Eindringen der Schneiden in den Knochen erleichtert wird, was wiederum zu einer besseren Schneidleistung führt. Die Spanbrechernuten benachbarter Zähne / Schneiden können zueinander versetzt ausgebildet und/oder angeordnet sein, insbesondere derart, dass die Nuten des einen Zahns an der den Schneiden des anderen Zahns entsprechenden Stellen befindlich sind und umgekehrt, so dass die Schneiden der Zähne jeweils abwechselnd am Abtrag beteiligt sind. Auf diese Weise kann die Größe und Form der abgetragenen Späne bestimmt werden, so dass ein Abtransport von abgetragenem Material optimiert werden kann. Außerdem wird auf diese Weise erreicht, dass bezogen auf den von dem jeweiligen Schneidenabschnitt bei dessen Rotation um die Drehachse überstrichenen Bereich jeweils nur eine Schneide in Eingriff ist, obwohl der Fräser nominell mehr Schneiden aufweist.

Die in Schneidrichtung rückwärtige Seite des Zahns, also der Zahnrücken kann insbesondere gegenüber der Rotationsachse geneigt ausgebildet und/oder angeordnet sein, so dass sich der Zahn von distal nach proximal konisch zum Schaft hin aufweitet. Der Zahnrücken kann insbesondere in Form einer ebenen Fläche ausgebildet sein, was die Zugänglichkeit, Reinigungseignung und Fertigungsfreundlichkeit begünstigt.

Die Zahndicke kann insbesondere mit einem Winkel von 1° bis 10° gegenüber der Rotationsachse von distal nach proximal ansteigen. Dieser Winkel wird als Zahndickenwinkel bezeichnet. Dies bewirkt eine besonders hohe Stabilität und außerdem eine gute Reinigungseignung. Die distale Dicke des Zahns bzw. der Schneide kann insbesondere zwischen 1/20 und 1/10 des Durchmessers des Fräserkopfs in radialer Richtung betragen. Auf diese Weise kann ein besonders großer Freiraum als Spanraum bei gleichzeitig hoher Stabilität von Zahn / Schneide zur Verfügung gestellt werden. Außerdem können durch eine entsprechende Ausbildung der Zahndicke die Schneidleistung und die Ratterneigung beeinflusst werden. Schließlich kann die Festigkeit des Fräserkopfs durch die Zahndicke in der jeweils erforderlichen Höhe definiert werden.

Eine weitere Aufführungsform der Erfindung ist dadurch gekennzeichnet, dass die Schneide einen konstanten Freiwinkel in einem Bereich zwischen 2° und 30° besitzt. Alternativ kann die Schneide einen Freiwinkel besitzen, der in einem Verlauf von distal nach proximal variiert, insbesondere in einem Bereich zwischen 2° und 30°. Außerdem kann die Schneide einen Spanwinkel von 0° besitzen. Der umlaufende Freiwinkel bildet quasi die den Materialabtrag definierende Form des Fräserkopfs aus. Durch eine entsprechende Ausbildung des Freiwinkels können die Schneidleistung und die Ratterneigung beeinflusst werden. Durch die vorstehend beschriebene Variation des Freiwinkels über seinen Verlauf können die Schneidleistung und die Ratterneigung weiter positiv beeinflusst werden.

Eine besonders anwenderfreundliche Ausführungsform ist dadurch gekennzeichnet, dass der Fräserkopf an seinem distalen Ende eine Spitze mit einem Winkel von 110° bis 150° aufweist, wodurch eine besonders gute und einfache Zentrierung und damit Handhabung beim Einsatz sichergestellt werden kann, insbesondere beim axialen Arbeiten mit dem Fräswerkzeug. Dieser Winkel wird als Spitzenwinkel bezeichnet. Proximal an die Spitze anschließend kann die Schneide / der Zahn im weiteren Verlauf bogenförmig, insbesondere kreisförmig, ausgebildet sein.

Nach der Erfindung kann der Fräser entweder als rechtsdrehender Fräser (Rechtsschneider) oder als linksdrehender Fräser (Linksschneider) ausgebildet sein.

Zusammenfassend kann man sagen, dass die Erfindung einen chirurgischen Fräser zur Verfügung stellt, der insbesondere nur zwei Schneiden aufweisen kann. Der Zahnzwischenraum ist vorzugsweise derart gestaltet, dass das abgetragene Gewebe möglichst ungehindert abtransportiert werden kann. Die Zahnform kann insbesondere derart ausgebildet sein, dass alles Material, das nicht für die Festigkeit des Zahns erforderlich ist, entfernt ist. Außerdem können Oberflächen des Fräsers, insbesondere des Fräserkopfs, besonders einfach glatt und eben ausgebildet sein, was die Spanabfuhr begünstigt. Infolge der geringen Temperaturentwicklung und der besonders guten Spanabfuhr kann mit dem erfindungsgemäßen Fräser ein besonders schonender Materialabtrag erzielt werden, wobei Veränderungen und/oder Schädigungen des abgetragenen Material weitgehend, wenn nicht sogar vollständig vermieden werden. Mittels eines erfindungsgemäßen Fräsers abgetragenes Material eignet sich daher besonders gut für eine künstliche Nachzüchtung derartigen Materials im Rahmen des erfindungsgemäßen Verfahrens.

Die Erfindung bewirkt insbesondere die folgenden Vorteile:
- sehr gute Schneidleistung besonders in axialer Richtung
- sehr gute Spanabfuhr, da nur zwei Schneiden
- reduzierte Temperaturentwicklung an der Schneide und am Gewebe
- sehr gut zu reinigender Fräserkopf
- symmetrischer Aufbau der Schneiden
- gut zu reinigen, da sehr gut zugängliche Oberflächen
- kein negativer Spanwinkel
- geringe Ratterneigung
- keine Neigung zum Verstopfen der Zahnzwischenräume
- sehr ruhiger Lauf ohne Vibration, da die Masse symmetrisch zur Rotationsachse angeordnet ist
- infolge der geringen Anzahl an Zähnen / Schneiden kostengünstig herstellbar
- einfach und kostengünstig herzustellender Fräser für Highspeed-Anwendungen in der Medizin zum Bearbeiten von Knochen

Weitere Merkmale und Vorteile der vorliegenden Erfindung ergeben sich aus der folgenden beispielhaften und nicht beschränkenden Beschreibung der Figuren. Diese sind lediglich schematischer Natur und dienen nur dem Verständnis der Erfindung. Dabei zeigen:
Fig. 1 zwei perspektivische Ansichten eines Rosenfräsers nach dem Stand der Technik,
Fig. 2 eine perspektivische Ansicht eines Einschneiders nach dem Stand der Technik,
Fig. 3 eine perspektivische Teilansicht eines distalen Abschnitts eines Fräsers nach einer ersten Ausführungsform der Erfindung,
Fig. 4 eine seitliche Ansicht des Fräsers der Figur 3,
Fig. 5 eine Schnittansicht des Fräsers der Figur 3 in einer Richtung quer zur Rotationsachse,
Fig. 6 eine Aufsicht auf den Fräser der Figur 3,
Fig. 7 eine Vorderansicht des Fräsers der Figur 3,
Fig. 8 einen Teilschnitt des Fräsers der Figur 3 durch die Rotationsachse,
Fig. 9 eine perspektivische Ansicht des Fräsers der Figur 3 aus einer anderen Richtung,
Fig. 10 eine weitere perspektivische Ansicht des Fräsers der Figur 3 aus einer weiteren Richtung,
Fig. 11 eine perspektivische Ansicht einer anderen Ausführungsform eines Fräsers nach der Erfindung,
Fig. 12 eine seitliche Ansicht des Fräsers der Figur 11,
Fig. 13 eine Aufsicht auf den Fräser der Figur 11,
Fig. 14 eine Vorderansicht des Fräsers der Figur 11,
Fig. 15 eine perspektivische Ansicht einer anderen Ausführungsform eines Fräsers nach der Erfindung,
Fig. 16 eine perspektivische Ansicht des Fräsers der Figur 15 aus einer anderen Richtung,
Fig. 17 eine perspektivische Ansicht einer anderen Ausführungsform eines Fräsers nach der Erfindung,
Fig. 18 eine der Figur 7 entsprechende Darstellung mit einer Kennzeichnung der Spanräume,
Fig. 19 eine der Figur 14 entsprechende Darstellung mit einer Kennzeichnung der Spanräume,
Fig. 20 eine Aufsicht auf eine weitere Ausführungsform eines Fräsers nach der Erfindung,
Fig. 21 eine Schnittansicht des Fräsers der Figur 20 in einer Richtung quer zur Rotationsachse,
Fig. 22 eine Schnittansicht des Fräsers der Figuren 20 und 21 in einer seitlichen Ansicht,
Fig. 23 eine vergrößerte Detailansicht aus Fig. 22,
Fig. 24 eine seitliche Ansicht einer weiteren Ausführungsform eines Fräsers nach der Erfindung,
Fig. 25 eine Aufsicht auf den Fräser der Figur 24,
Fig. 26 eine Vorderansicht des Fräsers der Figuren 24 und 25 in einer Richtung quer zur Rotationsachse und
Fig. 27 eine vergrößerte Detailansicht aus Fig. 24.

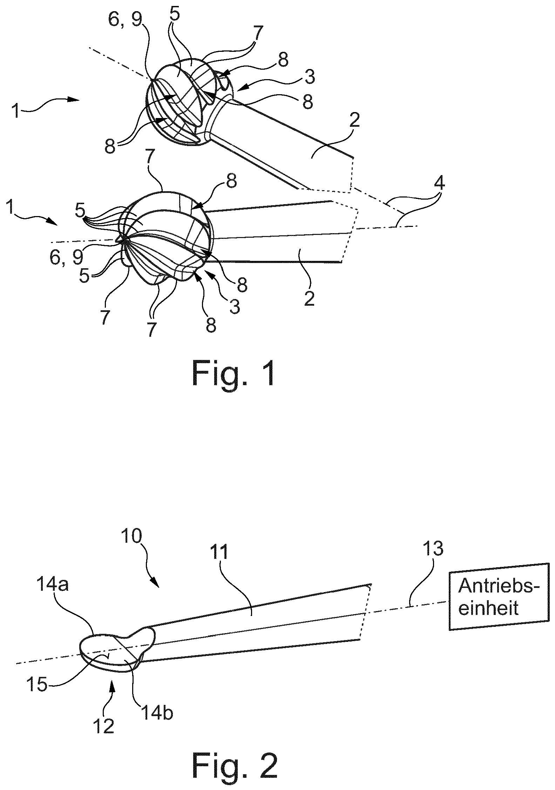

Figur 1 zeigt einen Rosenfräser 1 nach dem Stand der Technik in zwei perspektivischen Ansichten aus unterschiedlichen Blickrichtungen. Dieser weist proximal einen Fräserschaft 2 und distal einen Fräserkopf 3 mit einer annähernd kugelförmigen Außenkontur auf. Der Rosenfräser 1 besitzt eine Längsachse 4, die bei einem Betrieb zugleich seine Rotationsachse 4 ist. Er ist im dargestellten Beispiel mit insgesamt acht Zähnen 5 versehen, an deren von der Rotationsachse 4 abgewandten Seite jeweils eine von einer distalen Spitze 6 des Fräserkopfs 3 in Richtung proximal zum Fräserschaft 2 hin verlaufende Schneide 7 ausgebildet ist. Zwischen jeweils zueinander benachbarten Zähnen 5 ist jeweils ein Zahnzwischenraum 8 ausgebildet. Die Zähne 5 besitzen jeweils einen Drall und erstrecken sich wendelförmig von distal nach proximal. Man kann daher sagen, dass die Zähne 5 und deren Schneiden 7 am distalen Ende 9 des Fräserkopfs an der Rotationsachse 4 des Rosenfräsers 1 zusammenlaufen. In den beiden Ansichten der Figur 1 ist deutlich gezeigt, dass die Höhe der Zähne 5 zum distalen Ende 9 hin abnimmt und unmittelbar an der Rotationsachse 4 bzw. der Spitze 6 null ist. Anders ausgedrückt ist die Tiefe der Zahnzwischenräume am distalen Ende 9 bzw. an der Spitze 6 gleich null und nimmt in Richtung proximal zum Fräserschaft 2 hin zu. Der bei einem axialen Vorschub in Richtung der Längsachse 4 des Fräsers 1 für abgetragenes Material zur Verfügung stehende Spanraum ist daher nahe der Spitze 6 sehr klein und an der Spitze 6 nicht vorhanden, so dass es leicht zu einem Verstopfen der Zahnzwischenräume 8 verbunden mit den vorstehend erläuterten Nachteilen kommen kann.

Die Figur 2 zeigt einen weiteren bekannten Fräser 10 in Form eines sogenannten Einschneiders 10 in einer perspektivischen Darstellung. Dieser weist proximal einen Fräserschaft 11 und distal einen Fräserkopf 12 auf. Der Einschneider 10 besitzt eine Längsachse 13, die bei einem Betrieb zugleich seine Rotationsachse 13 ist. Der Fräserkopf 12 ist durch eine einseitig eingebrachte Abflachung bis auf die Rotationsachse 13 gebildet und weist eine Schneide 14b auf, an der eine im Wesentlichen ebene Brustfläche 15 ausgebildet ist. Auf der gegenüberliegenden Seite der Schneide 14b ist eine zurückgesetzte Kante 14a ausgebildet. Diese bildet einen Freiraum / eine Freifläche für die Schneide 14b. Der Fräser ist ausschließlich für einen Rechtslauf ausgebildet, schneidet also nur im Rechtslauf. Durch seine Gestalt befindet sich die gesamte Masse des Fräserkopfs 11 auf einer Seite der Rotationsachse 13, so dass die Massenverteilung des Einschneiders 10 unsymmetrisch ist, was mit den eingangs beschriebenen Nachteilen verbunden ist.

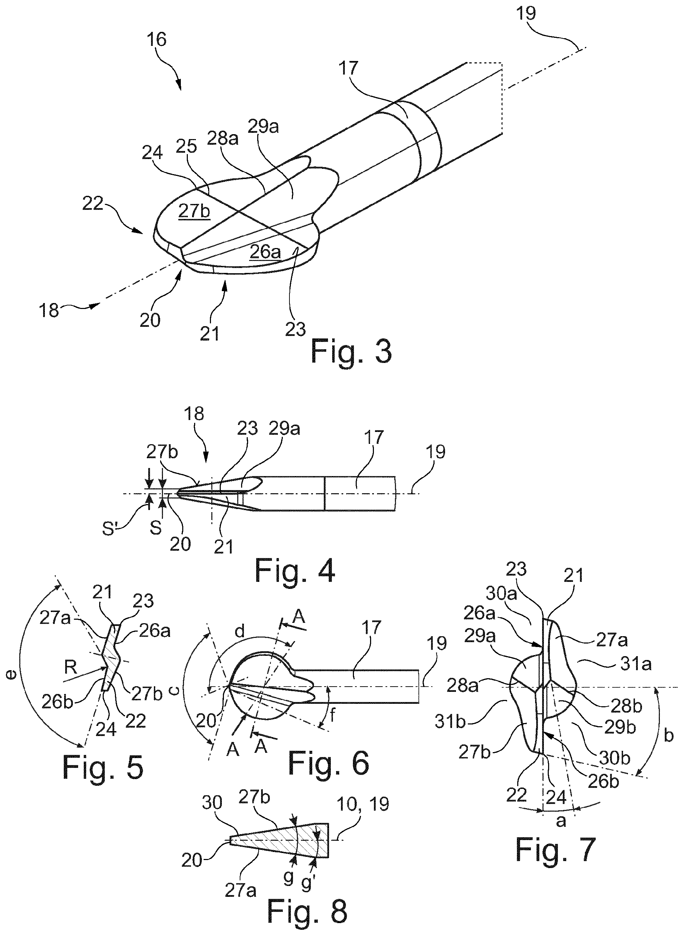

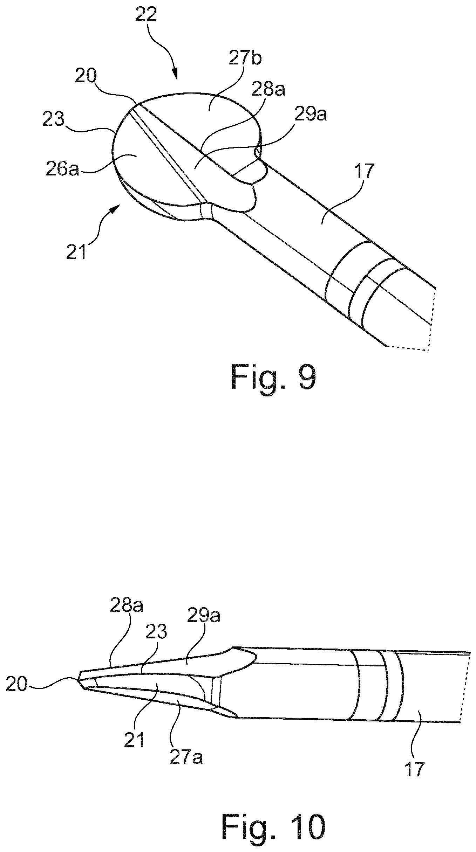

Die Figuren 3, 9 und 10 zeigen eine erste Ausführungsform eines chirurgischen Fräsers 16 nach der Erfindung, zu der in den Figuren 4 bis 8 außerdem Ansichten und Schnittansichten aus unterschiedlichen Richtungen gezeigt sind. Der Fräser 16 weist proximal einen Fräserschaft 17 und distal einen Fräserkopf 18 auf. Der Fräser 16 besitzt eine Längsachse 19, die bei einem Betrieb zugleich seine Rotationsachse 19 ist. Der Fräserschaft 17 erstreckt sich koaxial zur Rotationsachse 19, die den Fräserkopf 18 an dessen distalen Ende 20 zentral durchdringt (siehe insbesondere in Figur 7).

Der Fräserkopf 18 weist genau zwei Zähne 21, 22 auf, nämlich einen ersten Zahn 21 und einen zweiten Zahn 22. Der Zahn 21 besitzt eine Schneide 23 zur rotatorisch abtragenden Bearbeitung von Gewebe. Der Zahn 22 besitzt eine Schneide 24 zur rotatorisch abtragenden Bearbeitung von Gewebe. Die beiden Zähne 21, 22 und damit auch die Schneiden 23, 24 sind auf einander diametral gegenüberliegenden Seiten der Rotationsachse 19 des Fräsers 16 zueinander quer zur Rotationsachse 19 versetzt angeordnet. Die Schneiden 23, 24 sind jeweils an der von der Rotationsachse 19 abgewandten Seite des entsprechenden Zahns 21 bzw. 22 angeordnet und jeweils bogenförmig ausgebildet. Sie beginnen am distalen Ende 20 an der Rotationsachse 19 und erstrecken sich zunächst von distal-radial-innen nach proximal-radial-außen, um dann von der breitesten Stelle 25 des Fräserkopfs18 weiter bogenförmig nach proximal-radial-innen zum Fräserschaft 17 zu laufen. Die Schneiden 23, 24 sind jeweils ungewendelt und drallfrei ausgebildet. Durch diese Formgebung besitzt der Fräser 16 bei einer Rotation um seine Rotationsachse 19 eine Hüllbahn von im Wesentlichen kugelförmiger Form.

Die beiden Zähne 21, 22 sind identisch ausgebildet, so dass die Beschreibung des Zahns 21 in gleicher Weise auf den Zahn 22 zutrifft. Wie besonders gut in Figur 7 zu erkennen ist, besitzt der Zahn 21 eine ebene Brustfläche 26a, die die in Rotationsrichtung vordere Fläche des Zahns 21 bildet, sowie eine ebene Rückenfläche 27a, die die in Rotationsrichtung hintere Fläche des Zahns 21 bildet. Der Zahn 22 besitzt eine ebene Brustfläche 26b, die die in Rotationsrichtung vordere Fläche des Zahns 22 bildet, sowie eine ebene Rückenfläche 27b, die die in Rotationsrichtung hintere Fläche des Zahns 22 bildet. Die Rotationsrichtung ist die Schnittrichtung des Fräsers 16. Die Zähne 21, 22 sind relativ zueinander quer zur Rotationsachse 19 versetzt (siehe Figur 7), derart, dass die Brustflächen 26a, 26b der beiden Zähne 21, 22 eine gemeinsame Ebene aufspannen, in der die Rotationsachse 19 liegt. Insbesondere in Figur 7 ist gut zu erkennen, dass die Schneide 23 des ersten Zahns 21 und die Schneide 24 des zweiten Zahns 22 an der Spitze des distalen Endes 20 genau dort aufeinandertreffen, wo die Rotationsachse 19 das distale Ende 20 durchdringt. Die Rotationsachse 19 liegt in der Ebene der Brustfläche 26a und der Brustfläche 26b.

Die Rückenfläche 27a / 27b ist gegenüber der Brustfläche 26a /26b in der axialen Richtung um einen Zahnlängswinkel g' zwischen 4° und 12.5° geneigt, so dass der Fräserkopf 18 insgesamt einen Zahndickenwinkel g zwischen 8° und 25° (siehe Figur 8) besitzt. Außerdem ist die Rückenfläche 27a / 27b gegenüber der Brustfläche 26a /26b in einer Richtung quer zur Rotationsachse 19 um einen Zahnquerwinkel a zwischen 0° und 10° (siehe Figur 7) geneigt. Die Zahndicke S' des Zahns 21 am distalen Ende 20 ist durchmesserabhängig und beträgt etwa 1/10 (Ein Zehntel) des Durchmessers D des Fräserkopfs 18. Die Zahndicke S' des Zahns 22 am distalen Ende 20 ist ebenfalls durchmesserabhängig und beträgt etwa 1/10 (Ein Zehntel) des Durchmessers D des Fräserkopfs 18. Die Fräserkopfdicke S des Fräserkopfs 18 beträgt damit insgesamt etwa 2/10 des Durchmessers D (siehe Figur 4). Das distale Ende 20 des Fräserkopfs 18 ist als Spitze mit einem Spitzenwinkel c von 90° bis 150° ausgebildet (siehe Figur 6). Die Schneiden 23, 24 erstrecken sich jeweils von der Spitze am distalen Ende 20 über einen Freiflächenwinkel d von 110° bis 170° in Richtung des Fräserschafts 17 (siehe Figur 6).

Zwischen der Brustfläche 26a des Zahns 21 und der Rückenfläche 27b des Zahns 22 ist eine Stufe 28a oder Schulter 28a mit einer Schulterfläche 29a ausgebildet, die sich beginnend am distalen Ende 20 an der Rotationsachse 19 in Richtung proximal erstreckt. Zwischen der Brustfläche 26b des Zahns 22 und der Rückenfläche 27a des Zahns 21 ist eine Stufe 28b oder Schulter 28b mit einer Schulterfläche 29b ausgebildet, die sich beginnend am distalen Ende 20 an der Rotationsachse 19 in Richtung proximal erstreckt. Der Übergang von der Brustfläche 26a, 26b zur Schulterfläche 29a, 29b ist gerundet ausgebildet und ist in einem gegenüber der Rotationsachse 19 schrägen und in der Ebene der Brustfläche 26a, 26b liegenden Einstellwinkel f in Bereich von 2° bis 10° (siehe Figur 6) angeordnet. Die Schulterfläche 29a, 29b ist außerdem in einem in einer Ebene quer zur Rotationsachse 19 liegenden Zahnbrustwinkel e in Bereich von 110° bis 160° (siehe Figur 5) angeordnet. Der Freiwinkel b der jeweiligen Schneide 23, 24 liegt in einem Bereich von 0° bis 30° und ist umlaufend ausgebildet, das heißt über den gesamten Bereich des Freiflächenwinkels d (siehe Figur 7). Der Übergang von der Brustfläche 26a, 26b zur Schulterfläche 29a, 29b ist jeweils gerundet ausgebildet, was die Materialabfuhr und Reinigungseignung verbessert.

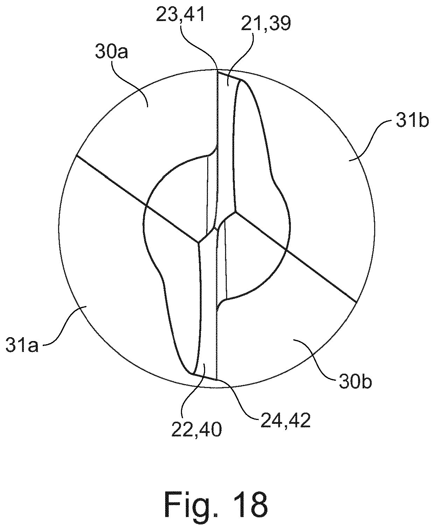

In Figur 7 ist gut zu erkennen, dass in Rotationsrichtung vor dem ersten Zahn 21 zwischen dessen Brustfläche 26a und dessen Schulterfläche 29a ein Spanraum 30a als Freiraum ausgebildet ist, der für mittels des ersten Zahns 21 des Fräsers 16 und dessen Schneide 23 abgetragenes Material zur Verfügung stellt und zusammen mit einem Bereich 31a hinter der Rückenfläche 27b des benachbarten Zahns 22 einen Gesamtspanraum 30a/31a bildet. Außerdem ist in Rotationsrichtung vor dem zweiten Zahn 22 zwischen dessen Brustfläche 26b und dessen Schulterfläche 29b ein Spanraum 30b als Freiraum ausgebildet ist, der für mittels des zweiten Zahns 22 des Fräsers 16 und dessen Schneide 24 abgetragenes Material zur Verfügung stellt und zusammen mit einem Bereich 31b hinter der Rückenfläche 27a des benachbarten Zahns 21 einen Gesamtspanraum 30b/31b bildet. Die Figur 18 zeigt die Ansicht der Figur 7 ohne Bezugszeichen, bei der die Freiräume 30a, 30b, die Bereiche 31a, 31b und die Spanräume 30a/31a, 30b/31b beider Zähne 21, 22 des Fräsers 16 mit entsprechender Schraffur (Spanräume 30a, 30b: schraffiert gekennzeichnet, Bereiche 31a, 31b: kariert gekennzeichnet) kenntlich gemacht sind. Aus Figur 7 geht außerdem hervor, dass sich der Spanraum 30a, 30b auf der der jeweiligen Schneide 23, 24 zugewandten Seite der Rotationsachse 19 von der Schneide 23, 24 bis in einen Bereich auf der der Schneide 23, 24 abgewandten Seite der Rotationsachse 19 erstreckt und dass jeder Spanraum 30a, 30b schneidenseitig der Rotationsachse 19 ausgebildet ist und sich zumindest in einem distalen Bereich auf die von der jeweiligen Schneide 23, 24 abgewandte Seite der Rotationsachse 19 erstreckt. Während in den Figuren 3 bis 10 zum ersten Zahn 21 gehörende Teile mit dem Bezugszeichenzusatz a gekennzeichnet sind, sind zum zweiten Zahn 22 gehörende Teile mit dem Bezugszeichenzusatz b kennzeichnet, beispielsweise ist die Brustfläche 26a die des ersten Zahns, während die Brustfläche 26b die des zweiten Zahns 22 ist.

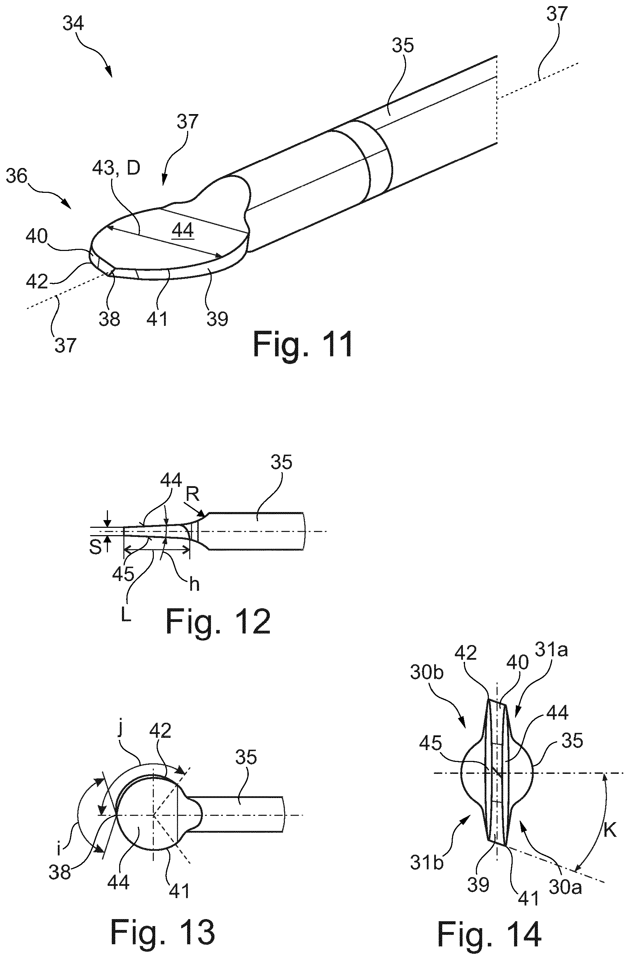

Die Figuren 11 bis 14 zeigen eine weitere Ausführungsform eines erfindungsgemäßen Fräsers 34. Der Fräser 34 weist proximal einen Fräserschaft 35 und distal einen Fräserkopf 36 auf. Der Fräser 34 besitzt eine Längsachse 37, die bei einem Betrieb zugleich seine Rotationsachse 37 ist. Der Fräserschaft 35 erstreckt sich koaxial zur Rotationsachse 37, die den Fräserkopf 36 an dessen distalen Ende 38 zentral durchdringt (siehe insbesondere in Figur 11).

Der Fräserkopf 36 weist einen ersten Zahn 39 und einen zweiten Zahn 40 auf. Der Zahn 39 besitzt eine Schneide 41 zur rotatorisch abtragenden Bearbeitung von Gewebe. Der Zahn 40 besitzt eine Schneide 42 zur rotatorisch abtragenden Bearbeitung von Gewebe. Die beiden Zähne 39, 40 und damit auch die Schneiden 41, 42 sind in einer gemeinsamen Ebene, in deren Zentrum die Rotationsachse 37 liegt, auf einander diametral gegenüberliegenden Seiten der Rotationsachse 37 des Fräsers 34 angeordnet. Die Schneiden 41, 42 sind jeweils an der von der Rotationsachse abgewandten Seite des entsprechenden Zahns 39 bzw. 40 angeordnet. Die Schneiden 41, 42 sind jeweils bogenförmig ausgebildet. Sie beginnen am distalen Ende 38 an der Rotationsachse 37 und erstrecken sich zunächst von distal-radial-innen nach proximal-radial-außen, um dann von der breitesten Stelle 43 des Fräserkopfs 34 weiter bogenförmig nach proximal-radial-innen zum Fräserschaft 35 zu laufen. Die Schneiden 41, 42 sind jeweils ungewendelt und drallfrei ausgebildet. Durch diese Formgebung der Schneiden 41, 42 bekommt der Fräser 34 bei einer Rotation um seine Rotationsachse 37 eine Hüllbahn von im Wesentlichen kugelförmiger Form.

Die beiden Zähne 39, 40 sind identisch ausgebildet, indem, wie besonders gut in Figuren 11 und 12 zu erkennen ist, auf einander diametral gegenüberliegenden Seiten der Rotationsachse 37 Abflachungen in den Fräserkopf 36 eingebracht sind. Der Fräserkopf 36 besitzt daher zwei einander gegenüberliegende, jeweils ebene Flächen 44, 45, die in einer Richtung quer zur Rotationsachse 37 zueinander parallel sind und in Richtung der Rotationsachse 37 zueinander geneigt sind. Die Flächen 44, 45 und die Schneiden 41, 42 sind derart zueinander angeordnet und ausgebildet, dass die Fläche 44 die Brustfläche der Schneide 41 und die Rückenfläche der Schneide 42, und die Fläche 45 die Brustfläche der Schneide 42 und die Rückenfläche der Schneide 41 bildet.

Die Fläche 44 ist gegenüber der Fläche 45 in der axialen Richtung um einen Zahndickenwinkel h zwischen 0° und 20° (siehe Figur 12) geneigt. Die Fräserkopfdicke S des Fräserkopfs 34 an der Spitze am distalen Ende 38 beträgt zwischen 0,05 mm und 1,0mm (siehe Figur 12). Das distale Ende 38 des Fräserkopfs 36 ist als Spitze mit einem Spitzenwinkel i von 90° bis 160° ausgebildet (siehe Figur 13). Die Schneiden 41, 42 erstrecken sich jeweils von der Spitze 43 am distalen Ende 38 über einen Freiflächenwinkel j von 110° bis 170° in Richtung des Fräserschafts 35 (siehe Figur 13). Der Fräserkopf 36 besitzt an der breitesten Stelle den Durchmesser D. Die Länge L des Konus, also die Erstreckung der Flächen 44, 45 in Richtung der Rotationsachse 37, beträgt etwa 7/9D (sieben-neuntel des Durchmessers D). Die Flächen 44, 45 gehen mit einem Radius R in den Fräserschaft 35 über, wobei der Radius R etwa dem halben Durchmesser D entspricht. Der Freiwinkel k der Schneiden 41, 42 liegt jeweils in einem Bereich von 0° bis 30° und ist umlaufend ausgebildet, das heißt über den gesamten Bereich des Freiflächenwinkels j (siehe Figuren 13 und 14).

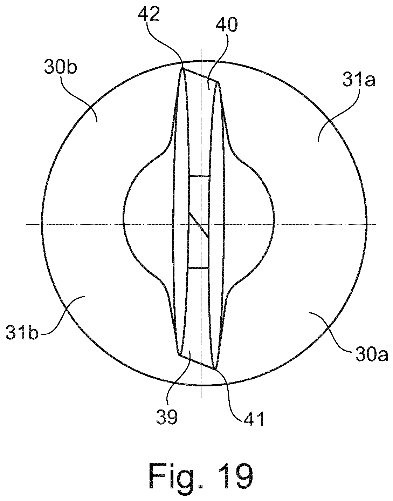

In Figur 14 ist gut zu erkennen, dass in Rotationsrichtung vor dem ersten Zahn 39 ein Spanraum 30a als Freiraum ausgebildet ist, der für mittels des ersten Zahns 39 des Fräsers 34 und dessen Schneide 41 abgetragenes Material zur Verfügung stellt und zusammen mit einem Bereich 31a hinter dem benachbarten Zahn 40 einen Gesamtspanraum 30a/31a bildet. Außerdem ist in Rotationsrichtung vor dem zweiten Zahn 40 ein Spanraum 30b als Freiraum ausgebildet ist, der für mittels des zweiten Zahns 40 des Fräsers 34 und dessen Schneide 42 abgetragenes Material zur Verfügung stellt und zusammen mit einem Bereich 31b hinter dem benachbarten Zahn 39 einen Gesamtspanraum 30b/31b bildet. Die Figur 19 zeigt die Ansicht der Figur 14 ohne Bezugszeichen, bei der die Freiräume 30a, 30b, die Bereiche 31a, 31b und Spanräume 30a/31a, 30b/31b beider Zähne 39, 40 des Fräsers 34 mit entsprechender Schraffur (Spanräume 30a, 30b: schraffiert gekennzeichnet, Bereiche 31a, 31b: kariert gekennzeichnet) kenntlich gemacht sind.

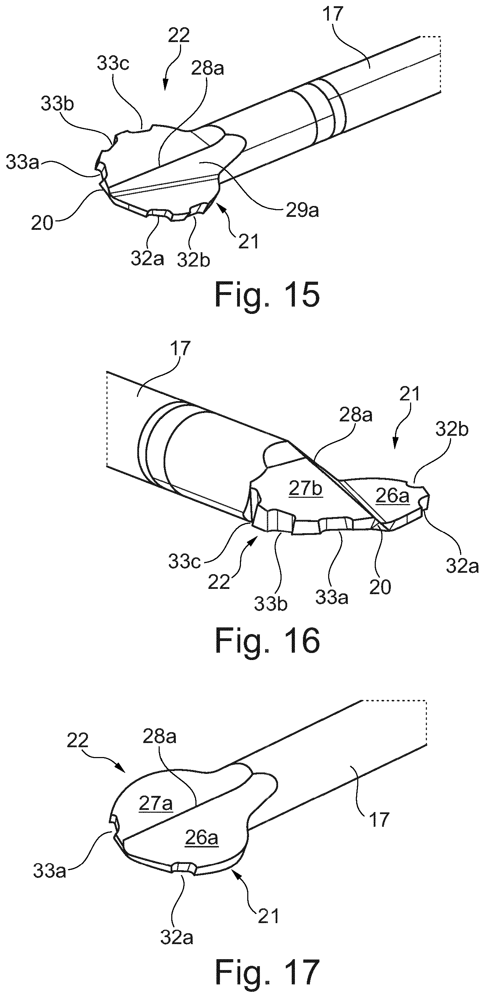

Die Figuren 15 und 16 zeigen eine Variante der vorstehend beschriebenen Ausführungsform der Figuren 3 bis 8, bei der die Schneide 23 des Zahns 21 Unterbrechungen in Form von Nuten 32a, 32b und die Schneide 24 des Zahns 22 Unterbrechungen in Form von Nuten 33a, 33b, 33c aufweisen. Die Nuten 32a und 32b sowie die Nuten 33a, 33b und 33c sind jeweils voneinander durch die zwischen ihnen verbliebenen Schneidenteile beabstandet. Sie sind außerdem in zentripetaler Richtung in den entsprechenden Zahn 21, 22 eingebracht und unterbrechen die Schneiden 23, 24. Die Nuten 32a und 32b des ersten Zahns 21 sind dabei relativ zu den Nuten 33a, 33b und 33c des zweiten Zahns 22 in umlaufender Richtung der Schneiden 23, 24 versetzt angeordnet, derart, dass an den Positionen, die den Nuten 32a, 32b des erstens Zahns 21 entsprechen, verbliebene Schneidenteile des zweiten Zahns 22 und an den Positionen, die den Nuten 33a, 33b, 33c des zweiten Zahns 22 entsprechen, verbliebene Schneidenteile des ersten Zahns 21 positioniert sind. Dies ermöglicht einen besonders gleichmäßigen Materialabtrag. Hinsichtlich der übrigen Merkmale gleichen sich die Ausführungsformen der Figuren 3 bis 8 und der Figuren 15 und 16. Es sei darauf hingewiesen, dass im Rahmen der Erfindung die Ausführungsform der Figuren 11 bis 14 mit derartigen Nuten 32a, 32b 33a, 33b, 33c versehen sein kann.

Die Figur 17 zeigt eine Variante der Ausführungsform der Figuren 15 und 16, bei der in jeder der Schneiden 23, 24 nur eine Nut 32a, 33a eingebracht ist. Dies ermöglicht einen besonders gleichmäßigen Materialabtrag. Hinsichtlich der übrigen Merkmale gleichen sich die Ausführungsformen der Figuren 3 bis 8 und der Figur 17. Es sei darauf hingewiesen, dass im Rahmen der Erfindung die Ausführungsform der Figuren 11 bis 14 mit derartigen Nuten 32a, 33a versehen sein kann.

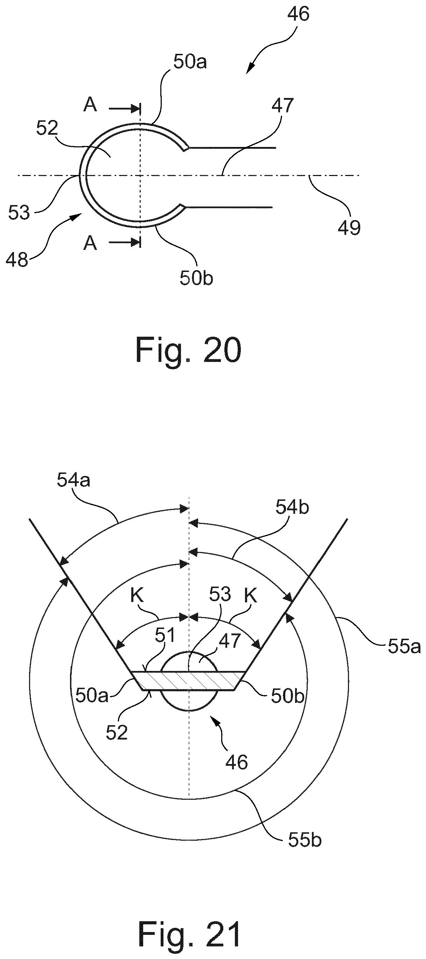

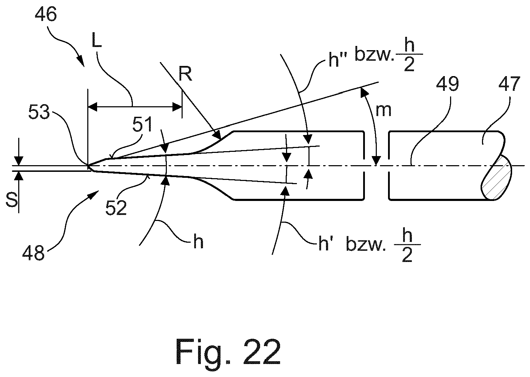

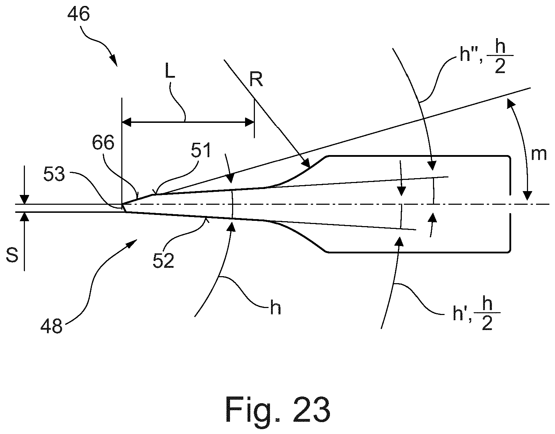

Die Figuren 20, 21, 22 und 23 zeigen eine weitere Ausführungsform eines erfindungsgemäßen Fräsers 46, der insbesondere von dem in Figur 2 gezeigten Einschneider 10 zu unterscheiden ist. Der Fräser 46 weist proximal einen Fräserschaft 47 und distal einen Fräserkopf 48 auf. Er besitzt außerdem eine Längsachse 49, die bei einem Betrieb zugleich seine Rotationsachse 49 ist. Der Fräserkopf 48 ist durch zwei symmetrisch einander gegenüberliegende Abflachungen bis jeweils kurz vor die Rotationsachse 49 gebildet, mit einer zusätzlichen Anspitzfläche 66, die in der Spitze 53 bis zur Rotationsachse 49 mit dem Freiwinkel m reicht, versehen und weist eine Schneide 50b auf, auf deren gegenüberliegenden Seite eine weitere Schneide 50a ausgebildet ist. Beide Schneiden 50a, 50b besitzen gleiche Form und Abmessungen, insbesondere gleiche Durchmesser, und bilden gemeinsam mit der Anspitzfläche 66 eine gemeinsame Brustfläche 51 aus. Auf der der Brustfläche gegenüberliegenden Seite bilden sie gemeinsam eine im Wesentlichen ebene Rückenfläche 52 aus. Wie insbesondere in den Figur 21 und 22 gezeigt ist, ist die Rückenfläche 52 kleiner als die Brustfläche 51, so dass an beiden Schneiden 50a, 50b jeweils ein Freiwinkel k ausgebildet ist. Die Brustfläche bildet für beide Schneiden 50a, 50b jeweils einen Freiraum / eine Freifläche aus. Die Vorderkante 53 des Fräserkopfs 48, die auch als Spitze 53 bezeichnet werden kann, ist vorzugsweise auch als Schneide ausgebildet, so dass eine Schnittwirkung in axialer Richtung, also in Richtung der Rotationsachse 49 bewirkt werden kann. Diese kann zum Beispiel dadurch erzielt werden, dass sich beide Schneiden 50a, 50b jeweils genau bis zur Rotationachse 49 erstrecken. Die Spanräume 54a, 54b und die Freiräume 55a, 55b beider Schneiden 50a, 50b sind in die Figur 21 eingezeichnet.

Der Fräser 46 ist damit sowohl für einen Rechtslauf wie auch für einen Linkslauf ausgebildet, schneidet also im Rechtslauf wie auch im Linkslauf. Durch seine Gestalt ist die Masse des Fräserkopfs 48 minimal durch den Wegfall der Anspitzung 66 unsymmetrisch zur Rotationsachse 49 und somit sehr laufruhig. Außerdem kann der Fräserkopf 48 mit überraschend kleinen Abmessungen ausgebildet sein, insbesondere mit einem Durchmesser kleiner als zum Beispiel 3mm, 4mm oder 5mm.

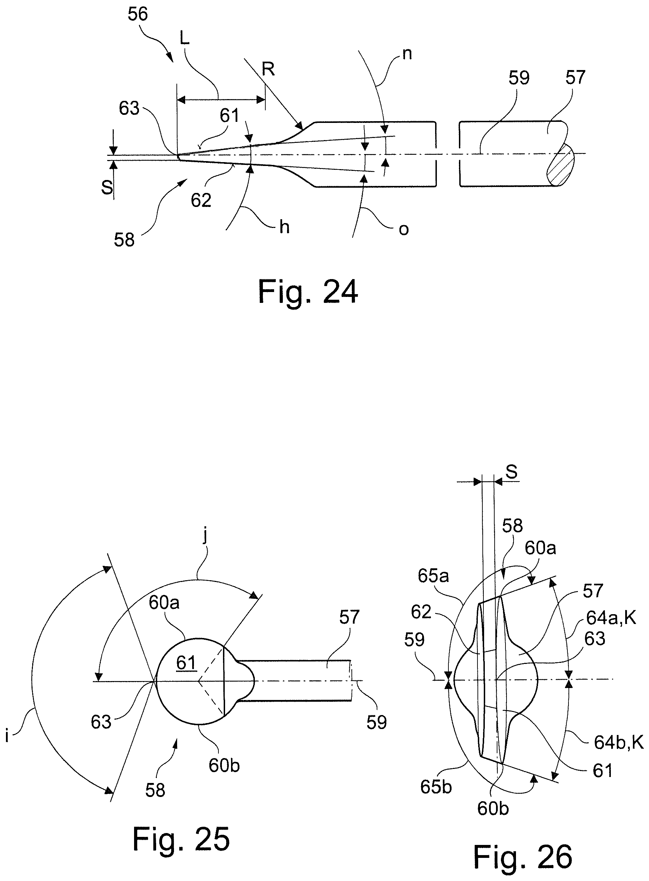

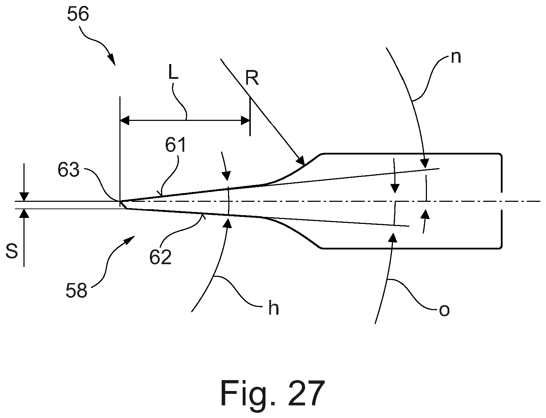

Die Figuren 24, 25, 26 und 27 zeigen eine weitere Ausführungsform eines erfindungsgemäßen Fräsers 56, der ebenfalls von dem in Figur 2 gezeigten Einschneider 10 zu unterscheiden ist. Der Fräser 56 ähnelt dem in den Figuren 20 und 21 gezeigten Fräser 46 und ist ebenfalls sowohl für einen Rechtslauf wie auch für einen Linkslauf ausgebildet, schneidet also im Rechtslauf wie auch im Linkslauf. Er weist proximal einen Fräserschaft 57 und distal einen Fräserkopf 58 auf. Er besitzt außerdem eine Längsachse 59, die bei einem Betrieb zugleich seine Rotationsachse 59 ist. Der Fräserkopf 58 ist durch zwei einander gegenüberliegende Abflachungen gebildet, wobei eine der Abflachungen eine Brustfläche 61 und die andere der Abflachungen eine Rückenfläche 62 ausbilden. Zwischen der Brustfläche 61 und der Rückenfläche 62 sind auf der einen Seite des Fräserkopfs 58 eine Schneide 60b und auf der gegenüberliegenden Seite eine weitere Schneide 60a ausgebildet. Das distale Ende des Fräserkopfs 58 ist in Form einer Spitze 63 ausgebildet. Das Besondere an dem Fräser 56 ist, dass dessen Brustfläche 61 an der Spitze 63 auf der Rotationsachse 59 liegt. Man kann sagen, dass die Brustfläche 61, die Schneiden 60a, 60b und die Rotationsachse 59 an der Spitze 63 zusammentreffen. Die beiden Schneiden 60a, 60b besitzen gleiche Form und Abmessungen, insbesondere gleiche Durchmesser. Wie insbesondere in Figur 24 gezeigt ist, ist die Rückenfläche 62 kleiner als die Brustfläche 61, so dass an beiden Schneiden 60a, 60b jeweils ein Freiwinkel m ausgebildet ist. Die Brustfläche bildet für beide Schneiden 60a, 60b jeweils einen Freiraum / eine Freifläche aus. Die Vorderkante oder Spitze 63 des Fräserkopfs 58 ist vorzugsweise auch als Schneide ausgebildet, so dass eine Schnittwirkung in axialer Richtung, also in Richtung der Rotationsachse 59 bewirkt werden kann. Die Spanräume 64a, 64b und die Freiräume 65a, 65b beider Schneiden 60a, 60b sind in die Figur 24 eingezeichnet.

Der Fräserkopf 58 besitzt an seiner Spitze 63 eine Fräserkopfdicke S. Ausgehend von der Spitze 63 sind die Brustfläche 61 und die Rückenfläche 62 um einen Zahndickenwinkel h zueinander geneigt. Außerdem ist die Brustfläche 61 gegenüber der Rotationsachse 59 um einen oberen Zahndickenwinkel n und die Rückenfläche 62 gegenüber der Rotationsachse 59 um einen unteren Zahndickenwinkel o geneigt, so dass die Beziehung h = n + o gilt.

Durch seine Gestalt besitzt der Fräser 56 eine etwas ungünstigere Massenverteilung hinsichtlich der Exzentrizität als der in den Figuren 20 und 21 dargestellte Fräser 46, allerdings besitzt er bessere Schneideigenschaften in Richtung der Rotationsachse 59. Auch kann der Fräserkopf 58 mit überraschend kleinen Abmessungen ausgebildet sein, insbesondere mit einem Durchmesser kleiner als zum Beispiel 3mm, 4mm oder 5mm.

### Bezugszeichenliste

- 1: Rosenfräser
- 2: Fräserschaft
- 3: Fräserkopf
- 4: Längsachse, Rotationsachse
- 5: Zähne
- 6: distale Spitze
- 7: Schneiden
- 8: Zahnzwischenraum
- 9: distales Ende
- 10: Einschneider
- 11: Fräserschaft
- 12: Fräserkopf
- 13: Längsachse, Rotationsachse
- 14a: zurückgesetzte Kante
- 14b: Schneide für Rechtslauf
- 15: Brustfläche
- 16: Fräser
- 17: Fräserschaft
- 18: Fräserkopf
- 19: Längsachse, Rotationsachse
- 20: distales Ende
- 21: erster Zahn
- 22: zweiter Zahn
- 23: Schneide
- 24: Schneide
- 25: breiteste Stelle
- 26a: Brustfläche des ersten Zahns 21, 39
- 26b: Brustfläche des zweiten Zahns 22, 40
- 27a: Rückenfläche des ersten Zahns 21, 39
- 27b: Rückenflache des zweiten Zahns 22, 40
- 28a: Stufe / Schulter des ersten Zahns 21, 39
- 28b: Stufe / Schulter des zweiten Zahns 22, 40
- 29a: Schulterfläche des ersten Zahns 21, 39
- 29b: Schulterfläche des zweiten Zahns 22, 40
- 30a: Spanraum des ersten Zahns 21, 39
- 30b: Spanraum des zweiten Zahns 22, 40
- 31a,b: Freiraum hinter Rückenflächen
- 32a,b: Nut
- 33a,b,c: Nut
- 34: Fräser
- 35: Fräserschaft
- 36: Fräserkopf
- 37: Längsachse, Rotationsachse
- 38: distales Ende
- 39: erster Zahn
- 40: zweiter Zahn
- 41: Schneide
- 42: Schneide
- 43: breiteste Stelle
- 44: Fläche
- 45: Fläche
- 46: Fräser
- 47: Fräserschaft
- 48: Fräserkopf
- 49: Längsachse, Rotationsachse
- 50a, 50b: Schneide
- 51: Brustfläche
- 52: Rückenfläche
- 53: Vorderkante, Spitze
- 54a, 54b: Spanraum
- 55a, 55b: Freiraum
- 56: Fräser
- 57: Fräserschaft
- 58: Fräserkopf
- 59: Längsachse, Rotationsachse
- 60a, 60b: Schneide
- 61: Brustfläche
- 62: Rückenfläche
- 63: Vorderkante, Spitze
- 64a, 64b: Spanraum
- 65a, 65b: Freiraum
- 66: Ausspitzfläche, Ausspitzung
- a: Zahnquerwinkel
- b: Freiwinkel
- c: Spitzenwinkel
- d: Freiflächenwinkel
- e: Zahnbrustwinkel
- f: Einstellwinkel
- g: Zahndickenwinkel
- g': Zahnlängswinkel
- h: Zahndickenwinkel
- i: Spitzenwinkel
- j: Freiflächenwinkel
- k: Freiwinkel
- L: Länge des Konus
- m: Ausspitzwinkel
- n R: oberer Zahndickenwinkel
- o: unterer Zahndickenwinkel
- D: Durchmesser
- R: Radius
- S: Fräserkopfdicke
- S': Zahndicke

## Patentansprüche

1. Chirurgischer Fräser (16, 34, 46, 56) mit einem Schaft (17, 35, 47, 57) zum drehangetriebenen Koppeln mit einer Antriebseinheit um eine sich in longitudinaler Richtung des Schafts (17, 35, 47, 57) erstreckende Rotationsachse (19, 37, 49, 59) und einem distal an dem Schaft (17, 35, 47, 57) angeordneten Fräserkopf (18, 36, 48, 58), wobei der Fräserkopf (18, 36, 48, 58) zumindest zwei Zähne (21, 22, 39, 40) mit jeweils Schneiden (23, 24, 41, 42, 50a, 50b, 60a, 60b) zur rotatorisch abtragenden Bearbeitung von Gewebe aufweist,
wobei die Schneiden (23, 24, 41, 42, 50a, 50b, 60a, 60b) jeweils zur abtragenden Bearbeitung von Gewebe sowohl in distaler Richtung als auch in lateraler Richtung ausgebildet sind,
wobei jeweils zwischen in umfänglicher Richtung benachbarten Zähnen (21, 22, 39, 40) ein Spanraum (30a, 30b, 54a, 54b) als Freiraum ausgebildet ist,
wobei jeder Spanraum (30a, 30b, 54a, 54b) sich auf der der jeweiligen Schneide (23, 24, 41, 42, 50a, 50b, 60a, 60b) zugewandten Seite der Rotationsachse (19, 37, 49, 59) von der Schneide (23, 24, 41, 42, 50a, 50b, 60a, 60b) bis in einen Bereich auf der der Schneide (23, 24, 41, 42, 50a, 50b, 60a, 60b) abgewandten Seite der Rotationsachse (19, 37, 49, 59) erstreckt,
**dadurch gekennzeichnet, dass**
die Zähne (21, 22, 39, 40) relativ zueinander quer zur Rotationsachse (19, 37) versetzt derart sind, dass Brustflächen (26a, 26b, 44, 45) der Zähne (21, 22, 39, 40) eine gemeinsame Ebene aufspannen, in der die Rotationsachse (19, 37) liegt.

2. Chirurgischer Fräser (16, 34, 46, 56) nach Anspruch 1, **dadurch gekennzeichnet, dass** die Schneiden (23, 24, 41, 42, 50a, 50b, 60a, 60b) jeweils bogenförmig von distal-radial-innen nach proximal-radial-außen ausgebildet sind und/oder jeweils ungewendelt und drallfrei ausgebildet sind.

3. Chirurgischer Fräser (16, 34, 46, 56) nach Anspruch 1 oder 2, **dadurch gekennzeichnet, dass** der Fräser (16, 34, 46, 56) genau zwei Zähne (21, 22, 39, 40) aufweist, die auf einander diametral gegenüberliegenden Seiten der Rotationsachse (19, 37, 49, 59) des Fräsers (16, 34, 46, 56) angeordnet sind.

4. Chirurgischer Fräser (16, 34, 46, 56) nach einem der vorstehenden Ansprüche, **dadurch gekennzeichnet, dass** die in Schnittrichtung vordere Fläche (26a, 26b, 44, 45, 51) des Zahns (21, 22, 39, 40) im Wesentlichen eben ausgebildet ist und/oder eine Stufe (28a, 28b) sich beginnend distal an der Rotationsachse (19, 37) in Richtung proximal erstreckt und relativ zur Rotationsachse (19, 37) in einem in der Ebene der Brustfläche (26a, 26b) liegenden Einstellwinkel (f) von 2° bis 10° angeordnet ist.

5. Chirurgischer Fräser (16, 34, 46, 56) nach einem der vorstehenden Ansprüche 1 bis 3, **dadurch gekennzeichnet, dass** die Zähne (21, 22, 39, 40) quer zur Rotationsachse (37) unversetzt einander diametral gegenüberliegen und insbesondere derart ausgebildet sind, dass eine Brustfläche (26a) des einen Zahns (39) und eine Rückenfläche (27b) des anderen Zahns (40) gemeinsam eine ebene Fläche (44) und die Rückenfläche (27a) des einen Zahns (39) und die Brustfläche (26b) des anderen Zahns (40) gemeinsam eine ebene Fläche (45) bilden.

6. Chirurgischer Fräser (16, 34, 46, 56) nach einem der vorstehenden Ansprüche, **dadurch gekennzeichnet, dass** zumindest eine der Schneiden (23, 24, 41, 42) wenigstens eine Unterbrechung (32a, 32b, 33a, 33b, 33c) oder Nut (32a, 32b, 33a, 33b, 33c) aufweist, die in zentripetaler Richtung in den Zahn (21, 22, 39, 40) eingebracht ist, eine Reduzierung der Schneidenlänge bewirkt und somit einer Verringerung der Vorschubkraft dient.

7. Chirurgischer Fräser (16, 34, 46, 56) nach einem der vorstehenden Ansprüche, **dadurch gekennzeichnet, dass** die Dicke des Zahns (21, 22, 39, 40) mit einem Zahndickenwinkel (g, h) von 1° bis 10° gegenüber der Rotationsachse (19, 37) von distal nach proximal ansteigt.

8. Chirurgischer Fräser (16, 34, 46, 56) nach einem der vorstehenden Ansprüche, **dadurch gekennzeichnet, dass** die distale Dicke des Zahns (21, 22, 39, 40) zwischen 1/20 und 1/10 des Durchmessers (D) des Fräserkopfs (18, 36) in radialer Richtung beträgt.

9. Chirurgischer Fräser (16, 34, 46, 56) nach einem der vorstehenden Ansprüche, **dadurch gekennzeichnet, dass** die Schneide (23, 24, 41, 42) einen entlang der Schneide (23, 24, 41, 42) konstanten Freiwinkel (b, k) von 2° bis 30° besitzt oder die Schneide (23, 24, 41, 42) einen Freiwinkel (b, k) in einem Bereich von 2° bis 30° besitzt, der entlang eines Verlaufs der Schneide (23, 24, 41, 42) variiert.

10. Chirurgischer Fräser (16) nach einem der vorstehenden Ansprüche, **dadurch gekennzeichnet, dass** die Schneide (23, 24) einen Spanwinkel von 0° besitzt.

11. Chirurgischer Fräser (16, 34, 46, 56) nach einem der vorstehenden Ansprüche, **dadurch gekennzeichnet, dass** der Fräserkopf (18, 36) an seinem distalen Ende (20, 38) eine Spitze mit einem Spitzenwinkel (c, i) von 110° bis 150° aufweist.

12. Chirurgischer Fräser (16, 34) nach einem der vorstehenden Ansprüche, **dadurch gekennzeichnet, dass** dieser rotationssymmetrisch zur Rotationsachse (19, 37) ausgebildet ist.

13. Chirurgischer Fräser (46, 56) nach einem der vorstehenden Ansprüche, **dadurch gekennzeichnet, dass** dieser eine erste Schneide (50a, 60a) aufweist, die einen Gewebeabtrag in einer ersten Drehrichtung bewirkt, und eine zweite Schneide (50b, 60b) aufweist, die einen Gewebeabtrag in einer der ersten Drehrichtung entgegengesetzten zweiten Drehrichtung bewirkt.

14. Chirurgischer Fräser (16, 34, 46, 56) nach einem der vorstehenden Ansprüche, **dadurch gekennzeichnet, dass** der Fräserkopf (18, 36, 48, 58) in einem Querschnitt durch die Rotationsachse (19, 37, 49, 59) eine flammenförmige, kegelförmige, olivenförmige oder walzenförmige Querschnittsform besitzt.

## Claims

1. A surgical milling cutter (16, 34, 46, 56) having a shaft (17, 35, 47, 57) for rotationally driven coupling with a drive unit about a rotational axis (19, 37, 49, 59) extending in a longitudinal direction of the shaft (17, 35, 47, 57) and a cutter head (18, 36, 48, 58) arranged distally on the shaft (17, 35, 47, 57),
the cutter head (18, 36, 48, 58) having at least two teeth (21, 22, 39, 40) with respective cutting edges (23, 24, 41, 42, 50a, 50b, 60a, 60b) for rotationally removing tissue,
the cutting edges (23, 24, 41, 42, 50a, 50b, 60a, 60b) each being designed for the removal of tissue both in the distal direction and in the lateral direction,
a chip space (30a, 30b, 54a, 54b) being formed as clearance in each case between adjacent teeth (21, 22, 39, 40) in the circumferential direction,
wherein each chip space (30a, 30b, 54a, 54b) extends on the side of the rotational axis (19, 37, 49, 59) facing the respective cutting edge (23, 24, 41, 42, 50a, 50b, 60a, 60b) from the cutting edge (23, 24, 41, 42, 50a, 50b, 60a, 60b) into a region on the side of the rotational axis (19, 37, 49, 59) facing away from the cutting edge (23, 24, 41, 42, 50a, 50b, 60a, 60b),
**characterized in that**
the teeth (21, 22, 39, 40) are offset relative to each other transversely to the rotational axis (19, 37) in such a way that front surfaces (26a, 26b, 44, 45) of the teeth (21, 22, 39, 40) span a common plane in which the rotational axis (19, 37) lies.

2. The surgical milling cutter (16, 34, 46, 56) according to claim 1, **characterized in that** the cutting edges (23, 24, 41, 42, 50a, 50b, 60a, 60b) are each arcuate from distal-radial-inward to proximal-radial-outward and/or are each non-helical and twist-free.

3. The surgical milling cutter (16, 34, 46, 56) according to claim 1 or 2,
**characterized in that** the milling cutter (16, 34, 46, 56) has exactly two teeth (21, 22, 39, 40) arranged on diametrically opposite sides of the rotational axis (19, 37, 49, 59) of the milling cutter (16, 34, 46, 56).

4. The surgical milling cutter (16, 34, 46, 56) according to any of the preceding claims, **characterized in that** the front surface (26a, 26b, 44, 45, 51) of the tooth (21, 22, 39, 40) in the cutting direction is substantially flat and/or a step (28a, 28b) extending in the proximal direction starting at the distal part at the rotational axis (19, 37) and is arranged relative to the rotational axis (19, 37) at a setting angle (f) of 2° to 10° lying in the plane of the front surface (26a, 26b).

5. The surgical milling cutter (16, 34, 46, 56) according to any of the preceding claims, **characterized in that** the teeth (21, 22, 39, 40) lie transversely to the rotational axis (37) diametrically opposite each other without offset and in particular are formed in such a way that a front surface (26a) of one tooth (39) and a rear surface (27b) of the other tooth (40) together form a flat surface (44), and the rear surface (27a) of one tooth (39) and the front surface (26b) of the other tooth (40) together form a flat surface (45).

6. The surgical milling cutter (16, 34, 46, 56) according to any of the preceding claims, **characterized in that** at least one of the cutting edges (23, 24, 41, 42) has at least one interruption (32a, 32b, 33a, 33b, 33c) or flute (32a, 32b, 33a, 33b, 33c), which is incorporated into the tooth (21, 22, 39, 40) in the centripetal direction, causes a reduction in the length of the cutting edges and thus serves to reduce the feed force.

7. The surgical milling cutter (16, 34, 46, 56) according to any of the preceding claims, **characterized in that** the thickness of the tooth (21, 22, 39, 40) increases with a tooth thickness angle (g, h) of 1° to 10° relative to the rotational axis (19, 37) from distal to proximal.

8. The surgical milling cutter (16, 34, 46, 56) according to any of the preceding claims, **characterized in that** the distal thickness of the tooth (21, 22, 39, 40) is between 1/20 and 1/10 of the diameter (D) of the cutter head (18, 36) in the radial direction.

9. The surgical milling cutter (16, 34, 46, 56) according to any of the preceding claims, **characterized in that** the cutting edge (23, 24, 41, 42) has a constant relief angle (b, k) of 2° to 30° along the cutting edge (23, 24, 41, 42) or the cutting edge (23, 24, 41, 42) has a relief angle (b, k) in a range of 2° to 30° that varies along a course of the cutting edge (23, 24, 41, 42).

10. The surgical milling cutter (16) according to any of the preceding claims,
**characterized in that** the cutting edge (23, 24) has a rake angle of 0°.

11. The surgical milling cutter (16, 34, 46, 56) according to any of the preceding claims, **characterized in that** the cutter head (18, 36) has a tip with a tip angle (c, i) of 110° to 150° at its distal end (20, 38).

12. The surgical milling cutter (16, 34) according to any of the preceding claims,
**characterized in that** it is rotationally symmetrical to the rotational axis (19, 37).

13. The surgical milling cutter (46, 56) according to any of the preceding claims,
**characterized in that** it has a first cutting edge (50a, 60a) which effects tissue removal in a first direction of rotation, and has a second cutting edge (50b, 60b) which effects tissue removal in a second direction of rotation opposite to the first direction of rotation.

14. The surgical milling cutter (16, 34, 46, 56) according to any of the preceding claims, **characterized in that** the cutter head (18, 36, 48, 58) has a flame-shaped, coneshaped, olive-shaped or roller-shaped cross-sectional shape in a cross-section through the rotational axis (19, 37, 49, 59).

## Revendications

1. Fraise chirurgicale (16, 34, 46, 56) avec un arbre (17, 35, 47, 57) pour le couplage entraîné en rotation avec une unité d'entraînement autour d'un axe de rotation (19, 37, 49, 59) s'étendant dans le sens longitudinal de l'arbre (17, 35, 47, 57) et une tête de fraise (18, 36, 48, 58) agencée distalement au niveau de l'arbre (17, 35, 47, 57), dans laquelle la tête de fraise (18, 36, 48, 58) présente au moins deux dents (21,22, 39, 40) avec respectivement des tranchants (23, 24, 41, 42, 50a, 50b, 60a, 60b) pour l'usinage par enlèvement rotatif de tissu,
dans laquelle les tranchants (23, 24, 41,42, 50a, 50b, 60a, 60b) sont formés respectivement pour l'usinage par enlèvement de tissu non seulement dans la direction distale mais aussi dans la direction latérale,
dans laquelle un espace pour copeaux (30a, 30b, 54a, 54b) est configuré comme espace libre respectivement entre des dents (21, 22, 39, 40) contigües dans le sens circonférentiel,
dans laquelle chaque espace pour copeaux (30a, 30b, 54a, 54b) s'étend sur le côté tourné vers le tranchant respectif (23, 24, 41, 42, 50a, 50b, 60a, 60b) de l'axe de rotation (19, 37, 49, 59) du tranchant (23, 24, 41,42, 50a, 50b, 60a, 60b) jusque dans une zone sur le côté éloigné du tranchant (23, 24, 41,42, 50a, 50b, 60a, 60b) de l'axe de rotation (19, 37, 49, 59),
**caractérisée en ce que**
les dents (21, 22, 39, 40) sont en départ les unes par rapport aux autres transversalement à l'axe de rotation (19, 37) de telle manière que des surfaces frontales (26a, 26b, 44, 45) des dents (21,22, 39, 40) définissent un plan commun dans lequel l'axe de rotation (19, 37) se trouve.

2. Fraise chirurgicale (16, 34, 46, 56) selon la revendication 1, **caractérisée en ce que** les tranchants (23, 24, 41,42, 50a, 50b, 60a, 60b) sont configurés respectivement en forme d'arc de la direction distale-radiale-vers l'intérieur à la direction proximale-radiale-vers l'extérieur et/ou sont configurés respectivement non enroulés et sans torsion.

3. Fraise chirurgicale (16, 34, 46, 56) selon la revendication 1 ou 2, **caractérisée en ce que** la fraise (16, 34, 46, 56) présente précisément deux dents (21, 22, 39, 40) qui sont agencées sur des côtés diamétralement opposés l'un à l'autre de l'axe de rotation (19, 37, 49, 59) de la fraise (16, 34, 46, 56).

4. Fraise chirurgicale (16, 34, 46, 56) selon l'une quelconque des revendications précédentes, **caractérisée en ce que** la surface avant dans le sens de coupe (26a, 26b, 44, 45, 51) de la dent (21, 22, 39, 40) est configurée de manière sensiblement plane et/ou un niveau (28a, 28b) s'étend en partant distalement au niveau de l'axe de rotation (19, 37) dans le sens proximal et est agencé dans un angle de réglage (f) se trouvant dans le plan de la surface frontale (26a, 26b) de 2° à 10° par rapport à l'axe de rotation (19, 37).

5. Fraise chirurgicale (16, 34, 46, 56) selon l'une quelconque des revendications précédentes 1 à 3, **caractérisée en ce que** les dents (21, 22, 39, 40) sont diamétralement opposées les unes aux autres transversalement à l'axe de rotation (37) sans départ et sont configurées en particulier de telle manière qu'une surface frontale (26a) de l'une dent (39) et une surface arrière (27b) de l'autre dent (40) forment ensemble une surface plane (44), et la surface arrière (27a) de l'une dent (39) et la surface frontale (26b) de l'autre dent (40) forment ensemble une surface plane (45).

6. Fraise chirurgicale (16, 34, 46, 56) selon l'une quelconque des revendications précédentes, **caractérisée en ce qu'**au moins l'un des tranchants (23, 24, 41,42) présente au moins une interruption (32a, 32b, 33a, 33b, 33c) ou une rainure (32a, 32b, 33a, 33b, 33c) qui est introduite dans le sens centripète dans la dent (21,22, 39, 40), provoque une réduction de la longueur de tranchant et sert ainsi à diminuer la force de poussée.

7. Fraise chirurgicale (16, 34, 46, 56) selon l'une quelconque des revendications précédentes, **caractérisée en ce que** l'épaisseur de la dent (21, 22, 39, 40) augmente selon un angle d'épaisseur de dent (g, h) de 1° à 10° par rapport à l'axe de rotation (19, 37) du sens distal à proximal.

8. Fraise chirurgicale (16, 34, 46, 56) selon l'une quelconque des revendications précédentes, **caractérisée en ce que** l'épaisseur distale de la dent (21, 22, 39, 40) est comprise entre 1/20 et 1/10 du diamètre (D) de la tête de fraise (18, 36) dans le sens radial.

9. Fraise chirurgicale (16, 34, 46, 56) selon l'une quelconque des revendications précédentes, **caractérisée en ce que** le tranchant (23, 24, 41,42) possède un angle de dépouille (b, k) constant le long du tranchant (23, 24, 41,42) de 2° à 30° ou le tranchant (23, 24, 41,42) possède un angle de dépouille (b, k) dans une plage de 2° à 30° qui varie le long d'une étendue du tranchant (23, 24, 41,42).

10. Fraise chirurgicale (16) selon l'une quelconque des revendications précédentes, **caractérisée en ce que** le tranchant (23, 24) possède un angle de coupe de 0°.

11. Fraise chirurgicale (16, 34, 46, 56) selon l'une quelconque des revendications précédentes, **caractérisée en ce que** la tête de fraise (18, 36) présente au niveau de son extrémité distale (20, 38) une pointe avec un angle de pointe (c, i) de 110° à 150°.

12. Fraise chirurgicale (16, 34) selon l'une quelconque des revendications précédentes, **caractérisée en ce que** celle-ci est configurée de manière symétrique en rotation par rapport à l'axe de rotation (19, 37).

13. Fraise chirurgicale (46, 56) selon l'une quelconque des revendications précédentes, **caractérisée en ce que** celle-ci présente un premier tranchant (50a, 60a) qui provoque un enlèvement de tissu dans un premier sens de rotation et présente un second tranchant (50b, 60b) qui provoque un enlèvement de tissu dans un second sens de rotation opposé au premier sens de rotation.

14. Fraise chirurgicale (16, 34, 46, 56) selon l'une quelconque des revendications précédentes, **caractérisée en ce que** la tête de fraise (18, 36, 48, 58) possède une forme de section transversale en forme de flamme, de cône, d'olive ou de rouleau dans une section transversale passant par l'axe de rotation (19, 37, 49, 59).
